Europäisches Patentamt

**European Patent Office**

**Office européen des brevets**

(19)

(11) Publication number: **0 013 449**
**B1**

(12) **EUROPEAN PATENT SPECIFICATION**

(45) Date of publication of patent specification: **08.02.84**

(21) Application number: **79200774.2**

(22) Date of filing: **17.12.79**

(51) Int. Cl.³: **A 61 K 39/15,**
**A 61 K 39/295, C 12 N 7/00**

(54) **Vaccine against diseases caused by reo viruses, combined vaccine with Newcastle disease virus, process for preparing them and reo virus strain used for their preparation.**

(30) Priority: **20.12.78 NL 7812359**
**26.04.79 NL 7903299**

(43) Date of publication of application:
**23.07.80 Bulletin 80/15**

(45) Publication of the grant of the patent:
**08.02.84 Bulletin 84/6**

(84) Designated Contracting States:
**AT CH DE GB IT NL SE**

(56) References cited:
**GB - A - 1 324 619**
**NL - A - 7 408 579**

**AVIAN PATHOLOGY, vol. 6, 1977, pages 271-284 U.S.A. L. VAN DER HEIDE: "Viral arthritis/tensosynovitis: a review"**
**UNLISTED DRUGS, vol. 27, no. 7, 108P, 1975**

(73) Proprietor: **Gist - Brocades N.V.**
**Wateringseweg 1 P.O. Box 1**
**NL-2600 MA Delft (NL)**

(72) Inventor: **Apontoweil, Peter**
**Burg. Martenslaan 39**
**NL-3956 EK Leersum (NL)**
Inventor: **Krasselt, Manfred Max**
**Park Arenberg 59**
**NL-3731 EP De Bilt (NL)**

(74) Representative: **Van der Straaten, Jan Anthony et al,**
**c/o GIST-BROCADES N.V. Patents and Trademarks Department Wateringseweg 1 P.O. Box 1**
**NL-2600 MA Delft (NL)**

Courier Press, Leamington Spa, England.

## Vaccine against diseases caused by reo viruses, combined vaccine with Newcastle disease virus, process for preparing them and reo virus strain used for their preparation

The invention relates to vaccines against diseases caused by avian reo virus in poultry, occurring frequently in recent years and causing big economic losses in poultry farms. The invention also relates to combined vaccines against infections in poultry and to the preparation of the vaccines.

It is known that reo viruses may cause several diseases in poultry, such as arthritis/tenosynovitis, myocarditis, hepatitis, hydropericardium, and diseases in the digestive tract and/or the respiratory tracts.

Avian Pathology 6 (1977) pages 271 to 284, for example, gives a survey of various pathogenic microorganisms which may cause by themselves or in combination, arthritis/tenosynovitis, such as Staphylococcus, Salmonella, Pasteurella, Erysipelotrix, *Mycobacterium avium, Bacterium arthropyogenes, Escherichia venezuelensis, Streptobacillus moniliformis, Mycoplasma gallisepticum, Mycoplasma synoviae* and reo viruses, such as the Connecticut reo virus isolate S 1133, the LZ 671 virus, or the RAM-1 virus, the reo viruses being considered the principal disease inducers. It has been found that viral arthritis/tenosynovitis and other diseases caused by reo virus infection plays an important part in the cause of considerable economic losses in the poultry farming in many parts of the world.

Furthermore, there has been proposed a method for giving protection against viral arthritis by vaccination during the growth period and subsequent transfer of maternally produced anti-bodies to the descendants by means of vaccination with reo virus vaccines. It has been found experimentally in this connection that immunisation of breeding animals only protects the first generation descendants, but not the second generation descendants.

It is known from e.g. Avian Pathology no. 3, 7, 407—419 (1978) that a synergistic relation might exist between *M. synoviae* and reo virus. Although, as stated by the authors, synergism was not proved conclusively, the effect of simultaneous challenge with reo virus and *M. synoviae* on the appearance of several disease symptoms, such as inflammation of the synovial membrane, the tendon sheath particularly in the tarsometarsal region, myocarditis and especially growth inhibition, was significantly greater than could be expected from an addition of effects caused by the two organisms separately.

It also appears from prior publications, e.g. in Neth. J. Vet. Sci. Vol. 3, No. 1 (1970) pages 5 to 10, that reo virusses, such as the LZ 671 reo virus, are considered to be responsible predominantly for synovitis symptoms in broiler parents.

Moreover, Poultry Digest of February 1978, page 102, also discloses that reo virus may cause growth inhibition and diarrhoea in broilers, and that oral infections with digestive suspensions may cause growth inhibition in chickens. Although virus material cultivated in eggs did not cause growth inhibition after oral administration, but caused "pasting", intraperitoneal injection of virus cultivated in eggs caused growth inhibition. The observed disease symptoms are brought into relation with an apparently necessary combination of infectious agents.

On the other hand more recent publications disclose that the cause of rachitis-like symptoms may be primarily attributed to the occurrence of toxic compounds produced by moulds such as *Fusarium moniliforme* Sheldon.

Zbl. Vet. Med. B, 25 (1978) pages 29 to 44, disclose, for example, that rachitis symptoms observed during recent years in various countries in broilers appear to be caused primarily by toxic compounds produced by the above indicated mould, which appeared able to develop in the usual poultry feed from potentially present spores, preferably under the somewhat moist conditions of the autumn and winter seasons.

Although in the years preceding those in which the published symptoms occurred, the observed rachitis symptoms could reasonably be combated by a vitamin therapy, this therapy did not lead, or did lead substantially less to improvement of the disease symptoms found later on.

It has been tried, therefore, to reproduce the symptoms observed later on by administration of feed selected especially in this connection, and to influence those symptoms by administration of vitamins and/or minerals and to investigate those symptoms more deeply, avoiding as far as possible the occurrence of diseases caused by parasites or microorganisms pathogenic for poultry, such as reo viruses, *Mycoplasma gallisepticum* and *M. synoviae* and other bacteria, by means of processes suitable for that purpose.

It is concluded that, also in view of harmful properties of the metabolism products of especially *Fusarium monoliforme* found in recent years and the mentioned disease symptoms in broilers, there must be a causual connection, despite the fact that no accurate decisive identification of the metabolites actually responsible therefore has taken place.

From, e.g. Feedstuffs, May 8, 1978, pages 95 and 96, it appears that the actual main cause of those rachitis symptoms has actually not yet been found.

It was, however, found surprisingly that the still to a greater extent occurring aberrations in the skeleton, such as rachitis-like symptoms, osteoporose c.q. femur degeneration, in poultry, especially broilers, often occurring together with growth, inhibitions and diarrhoea, may be com- batted by vaccination of broiler mother animals and their descendants with inactivated vaccines derived from certain avian reo viruses. While it is known, however, from e.g. Avian Pathology 6 page 279 that attempts have been made in the past to inhibit viral arthritis/tenosynovitis in poultry by vaccination with reo virus vaccines, but it definitely was not obvious for an expert to use avian reo virus vaccines to combat, aberrations in the skeleton such as e.g. rachitis-like symptoms, osteoporose c.q. femur degeneration, which have become more and more serious in recent years in all parts of the world as a problem needing more and more urgently an effective and efficient solution, as both types of symptoms were held to have completely different origins by people skilled in the art.

The vaccination with reo virus vaccines proposed now is preferably applied to broiler chicken mother animals and preferably immediately before the lay by those animals, in order to avoid the above indicated aberrations of the skeleton and the diarrhoea and growth inhibition caused by avian reo viruses in broilers due to virus infections through the eggs and, in addition, to protect the descendants against this infection by maternal immunity transfer during the first weeks after birth.

The invention therefore relates to an inactivated vaccine against aberrations in the skeleton such as rachitis-like symptoms, osteoporose c.q. femur degeneration caused by avian reo viruses alone or in combination with others in poultry characterized in that it is derived from an aqueous reo virus liquid obtained by cultivation of the LZ 671 strain, as deposited on April 6, 1979 with the Collection d'Institut Pasteur, Paris under no. I.092.

The invention furthermore relates to an inactivated vaccine against diarrhoea and growth inhibition caused by avian reo virusses in poultry characterized in that it is derived from an aqueous reo virus liquid obtained by cultivation of the LZ 671 strain, as deposited on April 6, 1979 with the Collection d'Institut Pasteur, Paris under no. I.092.

The vaccines according to the invention may be obtained by a process characterized by the preparation of a reo virus-containing liquid by cultivating an avian reo virus strain LZ 671 deposited on April 6, 1979 with Collection d'Institut Pasteur, Paris under no. I.092 in a brooded chicken egg or in an SPF chicken embryo fibroblast cell culture in the form of a monolayer rolling bottle culture or a culture of cells attached to solid inert carriers, collecting the cultivated virus, inactivating it, suspending it in a buffered solution optionally homogenizing it and mixing it with an oily phase.

According to a preferred process for the preparation of a liquid containing a suitable reo virus such as the LZ 671 virus strain deposited on April 6, 1979 under I.092 with the Collection d'Institut Pasteur, Paris, the virus is cultivated in an SPF chicken embryo fibroblast cell culture, e.g. in a monolayer rolling bottle culture or a culture of cells attached to solid inert carriers, preferably in a monolayer rolling bottle culture.

Preferably use is made of a cell culture obtained by suspending the starting cells in a culture medium of basal Eagle medium with or without 5 to 15 volume parts of calf serum, about 0.22% by weight of sodium bicarbonate and 1 to 5 volume parts per total volume of a solution or suspension of one or more antibiotics such as penicillin G, streptomycin and natamycin, whereby the culture medium is separated when the cell culture is nearly closed.

The solution of antibiotics contains, e.g. 2 to $3 \times 10^4$ units of penicillin, 20 to 30 mg of streptomycin and 0.4 to 0.8 mg of natamycin per ml.

After inoculation with virus suspension the culture is incubated at 37°C for 0.5 to 3 hours to adhere the virus, and maintenance medium is added, consisting of Eagle's basal medium with 4 to 6 volume parts of calf serum, 3 to 6 parts of Tryptose phosphate broth (100 g/l), 10—20 parts of bovine amnion fluid and the additives mentioned herebefore, and the virus-containing medium and cells are harvested after 0.5 to 3 days after appearance of the CPE.

The cell culture is preferably inoculated with a reo virus suspension having an activity of $2 \times 10^6 - 10^8$ TCID 50/rolling culture bottle, preferably $30 \times 10^6$ TCID 50/rolling culture bottle.

The inactivation of the starting virus liquid may be carried out by means of the usual inactivators, e.g. with beta-propiolactone, combined or not combined with a stabiliser, or with formaldehyde (0.02—0.5%), more particularly by adding beta-propiolactone in a concentration of 0.05 to 0.25% by weight (calculated on the aqueous phase weight) to the buffered virus-containing liquid, and by incubating that liquid for 1 to 2 hours, preferably for 90 minutes at about 37°C. Then the virus liquid is homogenized in the usual way, if necessary. If desired, a preservative such as thiomersal or formalin in a buffered solution, may be added thereafter to the aqueous phase.

For the preparation of the ready for administration vaccine, 300 to 500 ml of an inactivated, optionally homogenized reo virus-containing liquid is added to 500 to 700 ml of an oily phase. The essential components of that oily phase are at least one oil selected from light paraffinic mineral oils (FDA quality), plant oils and naphthenic mineral oils, together with one or more suitable emulsifiers in the form of non-ionic surface-active compounds derived from alkylene oxides and/or hexahydric alcohols and/or higher natural fatty acids (10 to 20 carbon atoms), such as esters and ester-ethers.

Examples thereof are mannide monooleate (Span 80® and Arlacel A®) and polyoxyethylene (20) sorbitan monooleate (e.g. Tween 80®).

The volume ratio of the aqueous phase,

formed by the virus liquid, and the oily phase may vary from 7:13 to 3:7 and is preferably about 7:13.

It has been found that the aqueous phase must be added to the oily phase under vigorous stirring and/or homogenisation in order to obtain the desired stable thin-liquid final emulsion.

The oily phase contains 2 to 20% by weight (calculated on the oily phase weight) of emulsifier, preferably 2 to 15% by weight of Arlacel A® or Arlacel 80® or Span 80® and 0.2 to 4% of Tween 80®. The components of the oily phase are preferably heated separately in an autoclave to at least 110°C or sterily filtered as a mixture.

The invention also relates to inactivated virus vaccines derived from at least a reo virus-containing liquid, i.e. inactivated sole vaccines derived from reo viruses as well as combined inactivated vaccines ready for use and derived from reo virus and a Newcastle Disease Virus (hereinafter to be indicated by NDV). Such combined inactivated vaccines are to be applied against various diseases, which may be caused by reo viruses themselves, such as viral arthritis/tenosynovitis, myocarditis, hepatitis, hydropericardium, diseases in the digestive tract and/or respiratory tract, aberrations of the skeleton such as rachitis-like symptoms, osteoporose c.q. femur degeneration, diarrhoea and growth inhibition, and diseases mainly attributable to the action of other virus types, e.g. Newcastle disease.

It will be known to every expert that disease symptoms may often be caused by combinations of occurring virus types. In connection therewith poultry is, for practical reasons, simultaneously or in rapid succession, vaccinated with two or more virus vaccines against the most frequently occurring virus diseases, the hitherto usual method consisting of mixing standard volumes of different vaccines ready for use, and administering the vaccine dose composed on the spot, which thus generally consisted of twice the volume of the vaccines to be applied separately. In addition an accurate mixing cannot be or can hardly be guaranteed and problems may arise due to foreign infective compounds. Therefore it was aimed to provide an improved combined, ready for use vaccine giving sufficient protection against Newcastle disease as well as against one or more of the diseases caused by reo virus, such as viral arthritis/tenosynovitis, myocarditis, hepatitis, hydropericardium, diseases in the digestive tract and/or respiratory tract, aberrations of the skeleton, such as rachitis like symptoms, osteoporose c.q. femur degeneration, diarrhoea and growth inhibition.

Therefore the invention also relates to combined inactivated vaccines acting against Newcastle disease (ND) and reo virus infections at least derived from NDV and reo virus-containing liquids.

The Newcastle disease is still one of the most important respiration diseases in poultry and may cause a high rate of mortality in poultry of all ages, especially young broilers.

Various vaccines have been developed against the Newcastle disease, and it has turned out that these vaccines are preferably administered in the inactivated form, since that form provides a high rate of safety and results in high humoral anti-body titres especially with revaccinations. In addition the use of live vaccine sometimes caused distribution of the inoculation virus from vaccinated poultry to poultry susceptible to infections or not being immune.

Although various relatively safe and good immunizing live ND vaccines have been developed in the mean-time, as appears from British Patent Specification No. 1,510,100 there is still a clear need for inactivated ND vaccines.

Combined inactivated vaccines against respiratory diseases in poultry, characterized by the combination of a dead vaccine against infectious coryza, obtained by cultivating a *Haemophilus gallinarum* strain in a natural nutrient medium and by inactivating the bacteria, with a dead vaccine of an infectious bronchitis virus and a dead ND vaccine are known, e.g. from Dutch published patent Application No. 7117873 (published for opposition purposes under No. 157209).

U.S. Patent Specification No. 2,798,835 discloses combined vaccines consisting of a combination of a vaccine against infectious bronchitis and an ND vaccine, both components being derived from live viruses.

It is also known from UK Patent Application No. 1324619 to combine killed infectious bronchitis virus with killed ND virus. However, the vaccines of the present invention are derived from another combination of viruses, and involve mixing and homogenizing with an oily phase.

The invention relates therefore also to combined inactivated vaccine ready for use against symptoms in poultry caused by avian reo-viruses and by Newcastle disease virus (NDV) characterized in that the vaccine contains an oily phase and an aqueous phase, which aqueous phase comprises an aqueous reo-virus liquid and an aqueous liquid of an inactivated Newcastle disease virus (NDV).

The combined vaccine contains preferably an aqueous reo virus liquid which is obtained by cultivation of the LZ 671 strain, as deposited on April 6, 1979 with the Collection d'Institut Pasteur under No. I.092.

The combined, inactivated, ready for use vaccine against Newcastle disease and disease symptoms caused by reo virus is prepared by a process characterized in that an NDV-containing aqueous liquid obtained in a usual way is inactivated, is optionally homogenized and, together with an avian reo virus-strain, preferably LZ 671, containing aqueous liquid obtained by cultivating in a brooded chicken egg or in an SPF chicken embryo fibroblast cell culture in the form of a monolayer soiling bottle culture or a culture of cells attached to solid inert carriers being inactivated and optionally homogenized previously, is added to an oily phase containing, as essential components, at least one oil selected from light paraffinic mineral oils (FDA quality), plant oils and naphthenic mineral oils together with one or more emulsifiers in the form of non-ionic surface active compounds derived from alkylene oxides and/or hexahydric alcohols and/or higher fatty acids ($C_{10}$ to $C_{20}$), such as esters and ester-ethers, optionally a preserving agent being added to the aqueous phase.

According to the process for the preparation of the combined vaccines of the present invention an inactivated NDV-containing liquid is prepared according to a manner known per se, e.g. by cultivating the desired virus in an impregnated chicken egg, collecting the cultivated virus in a liquid of a titre usual for this kind of vaccine and inactivating, suspending the virus in a suitable buffered solution and/or homogenizing, and mixing with the inactivated reo virus liquid obtained by cultivating the virus according to methods described hereinbefore, collecting the formed virus in a liquid of a titre usable for this kind of vaccine and inactivating the virus liquid according to methods known per se.

For the preparation of NDV-containing liquid various, little virulent or practically avirulent, lentogenic or velogenic ND viruses having good immunizing properties may be used, such as the la Sota or Hitchner virus (lentogenic) or the Herts 33/56 virus (velogenic).

Specific examples of applicable virus strains are the strains P/76/5, P/76/4 and P/76/3 obtainable from Institut für Medizinische Mikrobiologie, Infections and Seuchenmedizin der Ludwig Maximilians Universität, München, the VR-108, VR-107, VR-109, VR-699, and VR-623 strains, obtainable from the American Type Culture Collection, Rockville, Maryland, USA, the Queensland V4 strain, obtainable from the Centr. Vet. Lab. Weybridge, Great Britain, or the LZ258P virus, deposited with the Collection d'Institut Pasteur, Paris under no. I-091.

For the preparation of the combined vaccines 150 to 250 ml of an NDV-containing liquid is inactivated, is optionally homogenized and, together with 100 to 250 ml of the reo virus-containing liquid, being inactivated and optionally homogenized first, is added to 500 to 700 ml of an oily phase of the composition mentioned hereinbefore. The virus-containing liquids have preferably been buffered.

The volume ratio of the NDV-containing liquid and the reo virus-containing liquid may vary from 3:4 to 5:1 and is preferably about 3:2.

The volume ratio of the aqueous phase, formed by the virus liquids, and the oily phase may vary from 7:13 and 3:7 and is preferably about 7:13.

The inactivation of both starting virus liquid components may be carried out with the usual inactivators, e.g. by means of beta-propiolactone optionally combined with a stabiliser, or formaldehyde. Preferably beta-propiolactone is added in a concentration of 0.05 to 0.25% by weight (calculated on the aqueous phase) to a buffered ND virus-containing liquid and the liquid is incubated at 37°C for 1 to 2 hours and preferably for 90 minutes, while the buffered reo virus liquid is also inactivated by means of beta-propiolactone optionally combined with a stabilizer in a concentration of 0.05—0.25% by weight of 37°C for 1 to 2 hours. Then the virus liquids are homogenized in the usual way, if necessary. A preserving agent may be added to the aqueous phase, such as thiomersal or formaline in a buffered solution.

For obtaining suitable combination vaccines with the desired good properties, a NDV liquid having a titre of $10^{9.8}$ to $10^{10.5}$EID 50/ml preferably $\geqslant 10^{10}$EID 50/ml and a reo virus liquid with a titre of $10^{5.5}$ to $10^{7.5}$EID 50 /ml, preferably $\geqslant 10^{6.0}$EID 50/ml must be used.

The vaccines prepared in this way are preferably administered to 1 to 30 weeks old, preferably 10 to 20 weeks old, and to broiler mother animals before their lay.

It will be appreciated, that the occurring immunologic response after application of the combined vaccines, found to be of at least the same magnitude as those ones caused by the separate virus vaccines could in no way expected or predicted by a person skilled in the art, as this immunologic response of the combined vaccine as compared to one or both separate single vaccines might decrease until an unacceptable level on account of a number of undesired interactions between the virus liquid components mutually or with one of the applied auxiliaries, which could not be previously excluded. Especially it might be expected that the applied beta-propiolactone should remain active for too long a time, as to one of the virus components, with reference to a less fast decomposition in the mixture.

The application of the combined vaccine of the present invention involves as advantage that a smaller volume pro time has to be administered, which causes less local irritations and the administration of a smaller amount of chemicals foreign to the body, or that less separate vaccinations are necessary. Moreover the earlier mentioned problems with reference to the mixing and the occurrence of foreign infectious matter are avoided.

The invention is illustrated by the following examples.

Example 1.
Preparation of reo virus vaccine
a) Culture of reo virus.
SPF eggs brooded for 10 days are inoculated with LZ 671 seed virus. After incubation at 37°C for 76 hours the AAL (amnium allantoic liquid) is harvested. The AAL contains then about $10^{6.0}$EID 50/ml. The virus suspension is frozen at −20°C and is stored for vaccine production.

b) Treatment of virus suspension.

The frozen virus suspension is defrosted and inactivated in a water bath with 0.05 to 0.25% beta-propiolactone at 37°C for 90 minutes. The suspension is kept overnight at +4°C. The inactivation is controlled by observation of the formation of CPE's on CEF followed by a hemabsorption test. Moreover previously brooded SPF chicken eggs are incubated with the inactivated virus fluid and are subsequently controlled.

The virus suspensions are homogenized by means of an Ultra Turrax homogenizer. The virus suspension is optionally diluted with PBS+0.3% of formalin, depending on the $TCID_{50}$ or the $EID_{50}$ of the AAF and the titre of the reo virus suspension.

c) Preparation of emulsion.

The virus suspension is injected in an oily phase at room temperature, while simultaneously homogenizing with the Ultra Turrax homogenizer. The addition of the aqueous phase is stopped when the ratio of 6.5 parts of oil to 3.5 parts of virus suspension is reached. Homogenizing is continued until the drop-size of the aqueous phase is about 0.05—0.5 um.

The oily phase used has the following composition:

| | |
|---|---|
| Marcol® 52 (white paraffinic Esso oil) | 93.6% |
| Arlacel® A or Arlacel® 80 or Span® 80 (mannide monooleate) | 6.0% |
| Tween® 80 (polyoxyethylene 20 sorbitan monooleate) | 0.4% |

The compounds of the oily phase are separately heated to 110°C in the autoclave or sterily filtered as a mixture.

A stable emulsion was obtained, tested by:

1) pipetting, directly after emulsifying, drops onto a water surface, whereby the drops do not spread, but remain in tact;

2) storing at 37°C for 4 weeks without formation of any aqueous phase. The vaccine obtained may be used in a dosage of 0.5 ml per animal intramuscularly in the breast or leg muscles or subcutaneously in the neck.

Example 2
Preparation of combined reo virus/NDV vaccine
a) Culture of reo virus (strain LZ 671).

From chicken eggs brooded for 10 days cell cultures are prepared in (Bellco) rolling culture bottles, starting from CEF in basal medium Eagle Diploid Earl's Powder (GIB Co., cat. No. 420—1300) with 10% by volume of newborn calf serum, while 0.22% by weight of NaHCO₃ and 2 volume-% of an antibiotic solution containing per ml $25 \times 10^3$ units of penicillin G, 25 mg of streptomycin and 0.6 mg of natamycin were added to the total volume.

The medium is sucked off when the cell cultures are nearly closed. Then the cultures are inoculated with LZ 671 virus suspension and filled up with basal medium Eagle containing 4 volume-% of calf serum, 4 volume-% of Tryptose phosphate broth (100 g/l) 15% bovine amnion fluid, 0.22% by weight of $NaHCO_3$ and 3% of the above mentioned antibiotic solution.

The reo virus-containing medium with the cells is harvested 1 day after appearance of CPE's.

The virus suspension is frozen at −20°C and is stored for vaccine production. A $TCID_{50}$ of this suspension is determined.

b) Cultivation of NDV

SPF eggs brooded for 11 days are inoculated with LZ 258P seed virus. After incubation at 37°C for 72 hours the AAF is harvested. The AAF contains then about $10^{10}$EID 50/ml.

The virus suspension is frozen at −20°C or lower and is stored for vaccine production.

c) Treatment of virus suspension.

The frozen virus suspensions are defrosted and inactivated in a water bath with 0.05 to 0.25% of beta-propiolactone at 37°C for 90 minutes. The suspensions are kept overnight at +4°C. The inactivation is controlled by testing the formation of CPE's on CEF or EID 50-test for the reo virus and EID 50-test for the NDV.

The virus suspensions are homogenized with an Ultra Turrax homogenizer. The suspension is optionally diluted with PBS+0.3% of formalin, depending on $TCID_{50}$ or $EID_{50}$.

The suspensions of the NDV and the reo virus are then mixed in a ratio of 6:4 and are used for preparation of the emulsion.

d) Preparation of the emulsion.

The mixed virus suspension is injected in the oily phase at room temperature, while simultaneously homogenizing with the Ultra Turrax homogenizer. The addition of the aqueous phase is stopped when the ratio of 6.5 parts of oil to 3.5 parts of virus suspension is reached. Homogenizing is continued until the drop-size of the aqueous phase is about 0.05—0.5 um.

The oily phase used has the following composition:

| | |
|---|---|
| Marcol® 52 (white paraffinic Esso oil) | 93.6% |
| Arlacel® A or Arlacel® 80 or Span® 80 (mannide monooleate) | 6.0% |
| Tween® 80 (polyoxyethylene 20 sorbitan monooleate) | 0.4% |

The components of the oily phase were heated separately to 110°C in an autoclave or sterily filtered as a mixture.

The final concentrations of the thus prepared emulsion are:

21% NDV containing buffered liquid and 14% reo virus containing buffered liquid. The vaccine obtained is used in a dosage of 0.5 ml per animal intramuscularly in the breast or leg muscles or subcutaneously in the neck. 10 days after vaccination antibodies are detected by the immuno diffusion test.

Example 3.
Preparation of combined reo/NDV vaccine.

a) A reo containing liquid is prepared according to the corresponding step of Example 1. The virus suspension is inactivated with 0.02—0.5% formaldehyde during 20 hours at 22°C and then homogenized by means of an Ultra Turrax homogenizer.

b) A NDV containing liquid is prepared according to the corresponding step of Example 2. The treatment of the NDV suspension is carried out in the same way as in Example 2.

c) The treated NDV-AAF and reo-AAF are mixed thereafter in a proportion of 5:4.

d) The combined virus suspensions are added to and mixed with an oily phase in the proportion of 6 parts of oily phase: 4 parts of virus containing liquid and an emulsion is prepared passing it through a colloid mill. The applied oily phase has the following composition:

| | |
|---|---|
| Marcol® 52 (white paraffinic Esso oil) | 91.4 vol% |
| Arlacel® A or Arlacel® 80 pr Span® 80 (mannide (monooleate) | 8.0 vol% |
| Tween® 80 (polyoxyethylene 20 sorbitan monooleate) | 0.6 vol% |

**Claims for the Contracting States: CH, DE, GB, IT, NL, SE**

1. Inactivated vaccine for use against aberrations in the skeleton such as rachitis-like symptoms, osteoporose c.q. femur degeneration caused by avian reo viruses alone or in combination with others in poultry characterized in that it is derived from an aqueous reo virus liquid obtained by cultivation of the LZ 671 strain, as deposited on April 6, 1979 with the Collection d'Institut Pasteur, Paris under no. I.092.

2. Inactivated vaccine for use against diarrhoea and growth inhibition caused by avian reo viruses in poultry characterized in that it is derived from an aqueous reo virus liquid obtained by cultivation of the LZ 671 strain, as deposited on April 6, 1979 with the Collection d'Institut Pasteur, Paris under no. I.092.

3. Combined inactivated vaccine ready for use against symptoms in poultry caused by avian reo viruses and by Newcastle disease virus (NDV), characterized in that the vaccine contains an inactivated Newcastle disease virus containing liquid, having a titre of $10^{9.8}$—$10^{10.5}$ $EID_{50}$/ml, and an inactivated reo virus containing liquid, having a titre of $10^{5.5}$—$10^{7.5}$ $EID_{50}$/ml, whereby the volume ratio between the Newcastle disease virus containing liquid and the reo virus containing liquid can vary from 3:4 to

5:1, and an oil phase having as essential components at least one oil selected from light paraffinic mineral oils (FDA quality), plant oils and naphthenic mineral oils, together with between 2 and 20% by weight of one or more suitable emulsifiers in the form of non-ionic surface-active compounds which are esters or ester-ethers derived from alkylene oxides and/or hexahydric alcohols and/or higher natural fatty acids (10 to 20 carbon atoms), whereby the volume ratio between the aqueous phase formed by both virus liquids and the oil phase can vary from 7:13 to 3:7.

4. Combined inactivated vaccine according to claim 3, characterized in that the aqueous reo virus liquid is obtained by .cultivation of the LZ 671 strain, as deposited on April 6, 1979 with the Collection d'Institut Pasteur under No. I.092.

5. Inactivated vaccine according to claim 1, characterized in that it contains an oily phase having as essential components at least one oil selected from light paraffinic mineral oils (FDA quality), plant oils and naphthenic mineral oils, together with between 2 and 20% by weight of one or more suitable emulsifiers in the form of non ionic surface-active compounds which are esters or ester-ethers derived from alkylene oxides and/or hexahydric alcohols and/or higher natural fatty acids (10 to 20 carbon atoms).

6. Inactivated vaccine according to claim 5, characterized in that the volume ratio of the aqueous phase, formed by the virus liquid, and the oily phase may vary from 7:13 to 3:7.

7. Inactivated vaccine according to claim 6, characterized in that the volume ratio of the aqueous phase, formed by the virus liquid and the oily phase is about 7:13.

8. Combined inactivated vaccine according to claim 3, characterized in that the volume ratio of the liquid containing the Newcastle disease virus and the liquid containing the reovirus is about 3:2.

9. Process for the preparation of an inactivated reo virus vaccine, characterized by the preparation of a reo virus-containing liquid by cultivating an avian reo virus strain LZ 671 deposited on April 6, 1979 with Collection d'Institut Pasteur, Paris under no. I.092 in a brooded chicken egg or in an SPF chicken embryo fibroblast cell culture in the form of a monolayer rolling bottle culture or a culture of cells attached to solid inert carriers, collecting the cultivated virus, inactivating it, suspending it in a buffered solution optionally homogenizing it and adding it under vigorous stirring and/or homogenizing to an oily phase.

10. Process according to claim 9, characterized in that as inactivator beta-propiolactone is added, optionally with a stabilizer.

11. Process according to claims 9 and 10, characterized in that as inactivator beta-propiolactone is added in a concentration of 0.05 to 0.25% by weight (calculated on the aqueous phase weight) to the virus containing liquid.

12. Process for the preparation of a combined, inactivated, ready for use vaccine against symptoms in poultry caused by avian reo virus and by Newcastle disease virus, characterized in that a Newcastle disease virus containing liquid, having a titre of $10^{9.8}$—$10^{10.5}$ $EID_{50}/ml$, is optionally homogenized, and together with an inactivated reo virus containing liquid, having a titre of $10^{5.5}$—$10^{7.5}$ $EID_{50}/ml$, whereby the volume ratio between the Newcastle disease virus containing liquid and the reo virus containing liquid can vary from 3:4 to 5:1, is added under vigorous stirring and/or homogenizing to an oil phase containing as essential components at least one oil selected from light paraffinic mineral oils (FDA quality), plant oils and naphthenic mineral oils, together with one or more suitable emulsifiers in the form of non-ionic surface-active compounds which are esters or ester-ethers derived from alkylene oxides and/or hexahydric alcohols and/or higher natural fatty acids (10 to 20 carbon atoms), whereby the volume ratio between the aqueous phase formed by both virus liquids and the oil phase can vary from 7:13 to 3:7, optionally a preservative being added to the aqueous phase.

13. Process according to claim 12, characterized in that the reo virus is of the strain LZ 671.

14. Process according to claim 13, characterized in that the volume ratio of the NDV containing liquid and the reo-virus containing liquid is about 3:2.

15. Reo virus strain LZ 671, deposited at April 6, 1979 with the Collection d'Institut Pasteur, Paris under no. I.092.

**Claims for the Contracting State: AT**

1. Process for the preparation of an inactivated reo virus vaccine characterized by the preparation of a reo virus-containing liquid by cultivating an avian reo-virus strain LZ 671, deposited on April 6, 1979 with the Collection d'Institut Pasteur, Paris, under no. I.092, in a brooded chicken egg or in an SPF chicken embryo fibroblast cell culture in the form of a monolayer rolling bottle culture of cells attached to solid inert carriers, collecting the cultivated virus, inactivating it, suspending it in a buffered solution and optionally homogenizing it and adding it under vigorous stirring and/or homogenizing to an oily phase.

2. Process according to claim 1, characterized in that the reo virus strain LZ 671 is cultivated in a cell culture in the form of a monolayer rolling bottle culture or cells attached to solid inert carriers of the starting cells in a culture medium of basal medium Eagle with optionally added thereto 5 to 15 parts by volume of calf serum, about 0.22% by weight of sodium bicarbonate, and 1 to 5 parts by volume of a solution or suspension of one or more antibiotics, preferably penicillin G, streptomycin and natamycin, the culture liquid is separated when the cell culture is nearly closed, is inoculated with the virus suspension, is incubated at 37°C for 0.5 to 3 hours in order to attach the virus, and a maintenance medium consisting of at least basal medium Eagle with 4 to 6 parts by volume of calf serum, 3 to 6 parts by volume of Tryptose phosphate broth (100 g/l), 10—20 parts by volume of bovine amnion fluid and the additives mentioned hereinbefore is added, and the virus containing medium and cells are harvested after 0.5 to 3 days after the appearance of the cytopathogenic effect (C.P.E.).

3. Process according to claim 1, characterized in that as inactivator beta-propiolactone is added in a concentration of 0.05 to 0.25% by weight (calculated on the aqueous phase weight) to the virus-containing liquid.

4. Process according to claim 1, characterized in that the virus-containing liquid is mixed with an oily phase, having as essential components at least one oil selected from light paraffinic mineral oils (FDA) quality), plant oils and naphthenic mineral oils, together with between 2 and 20% by weight of one or more suitable emulsifiers in the form of non-ionic surface-active compounds which are esters or ester-ethers derived from alkylene oxides and/or hexahydric alcohols and/or higher natural fatty acids ($C_{10}$ to $C_{20}$).

5. Process according to claim 1, characterized in that the volume ratio of the aqueous phase formed by the virus liquid and the oily phase may vary from 7:13 to 3:7.

6. Process according to claim 1, characterized in that the volume ratio of the aqueous phase formed by the virus liquid and the oily phase is about 7:13.

7. Process according to claim 1, characterized in that there is started with a reo virus-containing liquid having a titre of at least $10^{5.5}$ to $10^{7.5}$ $EID_{50}/ml$.

8. Process according to claim 1, characterized in that there is started with a reo virus-containing liquid having a titre of $10^{6.0}$ $EID_{50}/ml$.

9. Process for the preparation of a combined, inactivated, ready for use vaccine against symptoms in poultry caused by avian reo virus and by Newcastle disease virus, characterized in that a Newcastle disease virus containing liquid, having a titre of $10^{9.8}$—$10^{10.5}$ $EID_{50}/ml$, is optionally homogenized, and together with an inactivated reo virus containing liquid, having a titre of $10^{5.5}$—$10^{7.5}$ $EID_{50}/ml$, whereby the volume ratio between the Newcastle disease virus containing liquid and the reo virus containing liquid can vary from 3:4 to 5:1, is added under vigorous stirring and/or homogenizing to an oil phase containing as essential component at least one oil selected from light paraffinic mineral oils (FDA quality), plant oils and naphthenic mineral oils together with one or more suitable emulsifiers in the form of non-ionic surface-active compounds which are esters or ester-ethers derived from alkylene oxides and/or hexahydric alcohols and/or higher natural fatty acids (10 to 20 carbon atoms), whereby the volume ratio between the aqueous phase formed by both virus liquids and the oil phase can vary from 7:13 to 3:7, optionally a preservative being added to the aqueous phase.

10. Process according to claim 9, characterized in that the reo virus is of the strain LZ 671.

11. Process according to claim 9 and 10 characterized in that as inactivator beta-propiolactone is added in a concentration of 0.05 to 0.25% by weight (calculated on the aqueous phase weight) to the virus-containing liquid.

12. Process according to claim 9 and 10 characterized in that there is started with an NDV liquid having a titre of $10^{10}$ EID$_{50}$/ml.

13. Process according to claim 9 and 10 characterized in that there is started with a LZ 671 reo-virus liquid having a titre of $\geqslant 10_{6.0}$ EID$_{50}$/ml.

14. Process according to claim 9 and 10 characterized in that the volume ratio of the liquid containing Newcastle disease virus and the liquid containing the LZ 671 reo-virus liquid is about 3:2.

**Revendications pour les etats Contractants: CH(LI), DE, GB, IT, NL, SE**

1. Vaccin inactivé à utiliser contre les aberrations du squelette, comme les symptômes de type rachitique et l'ostéoporose, en l'occurrence la dégénérescence du fémur, provoquées par les reo virus aviaires isolément ou conjointement avec d'autres chez la volaille, caractérisé en ce qu'il dérive d'un liquide aqueux de reo virus obtenu par culture de la souche LZ 671 telle que déposée le 6 avril 1979 à la Collection de l'Institut Pasteur à Paris sous le n° I.092.

2. Vaccin inactivé à utiliser contre la diarrhée et l'inhibition de croissance provoquées par des reo virus aviaires chez la volaille, caractérisé en ce qu'il dérive d'un liquide aqueux de reo virus obtenu par culture de la souche LZ 671 telle que déposée le 6 avril 1979 à la Collection de l'Institut Pasteur à Paris sous le n° I.092.

3. Vaccin inactivé combiné prêt à l'administration contre les symptômes provoqués chez la volaille par des virus aviaires et par le virus de la maladie de Newcastle (NDV), caractérisé en ce que le vaccin contient un liquide contenant du virus de la maladie de Newcastle inactivé ayant un titre de $10^{9,8}$ à $10^{10,5}$ DI$_{50}$ d'embryon par ml, et un liquide contenant du reo virus inactivé ayant un titre de $10^{5,5}$ à $10^{7,5}$ DI$_{50}$ d'embryon par ml, le rapport en volume entre le liquide contenant du virus de la maladie de Newcastle et le liquide contenant du reo virus pouvant varier de 3:4 à 5:1, et une phase huileuse comprenant comme constituants essentiels au moins une huile choisie parmi les huile minérales paraffiniques légères (qualité FDA), les huiles végétales et les huiles minérales naphténiques, conjointement avec 2 à 20% en poids d'un ou plusieurs émulsionnants appropriés sous la forme de composés surfactifs non ioniques, qui sont des esters ou ester-éthers issus d'oxydes d'alcoylène et/ou d'alcools hexahydroxylés et/ou d'acides gras naturels supérieurs (10 à 20 atomes de carbone), étant entendu que le rapport en volume entre la phase aqueuse formée par les deux liquides viraux et la phase huileuse peut varier de 7:13 à 3:7.

4. Vaccin inactivé combiné suivant la revendication 3, caractérisé en ce que le liquide aqueux de reo virus est obtenu par culture de la souche LZ 671 telle que déposée le 6 avril 1979 à la Collection de l'Institut Pasteur à Paris sous le n° I.092.

5. Vaccin inactivé suivant la revendication 1, caractérisé en ce qu'il contient une phase huileuse comprenant comme constituants essentiels au moins une huile choisie parmi les huiles minérales paraffiniques légères (qualité FDA), les huiles végétales et les huiles minérales naphténiques, conjointement avec 2 à 20% en poids d'un ou plusieurs émulsionnants appropriés sous la forme de composés surfactifs non ioniques, qui sont des esters ou ester-éthers issus d'oxydes d'alcoylène et/ou d'alcools hexahydroxylés et/ou d'acides gras naturels supérieurs (10 à 20 atomes de carbone).

6. Vaccin inactivé suivant la revendication 5, caractérisé en ce que le rapport en volume de la phase aqueuse formée par le liquide viral à la phase huileuse peut varier de 7:13 à 3:7.

7. Vaccin inactivé suivant la revendication 6, caractérisé en ce que le rapport en volume de la phase aqueuse formée par le liquide viral à la phase huileuse est d'environ 7:13.

8. Vaccin inactivé combiné suivant la revendication 3, caractérisé en ce que le rapport en volume du liquide contentant le virus de la maladie de Newcastle au liquide contenant le reo virus est d'environ 3:2.

9. Procédé de préparation d'un vaccin de reo virus inactivé, caractérisé en ce qu'on prépare un liquide contenant du reo virus en cultivant une souche de reo virus aviaire LZ 671 déposée le 6 avril 1979 à la Collection de l'Institut Pasteur à Paris sous le n° I.092 dans un oeuf de poule embryonné ou dans un culture de fibroblastes d'embryon de poussin exempts d'agents pathogènes spécifiés sous la forme d'une culture en couche monocellulaire en flacon roulant ou d'une culture de cellules fixées sur des supports solides inertes, en collectant le virus cultivé, en l'inactivant, en le mettant en suspension dans une solution tamponnée, éventuellement en l'homogénéisant et en l'ajoutant sous vive agitation et/ou homogénéisation à une phase huileuse.

10. Procédé suivant la revendication 9, caractérisé en ce qu'on ajoute comme inactivateur de la bêta-propiolactone, éventuellement avec un stabilisant.

11. Procédé suivant les revendications 9 et 10, caractérisé en ce qu'on ajoute comme inactivateur de la bêta-propiolactone en une concentration de 0,05 à 0,25% en poids (à calculer sur le poids de la phase aqueuse) au liquide contenant le virus.

12. Procédé de préparation d'un virus combiné inactivé prêt à l'administration contre les symptômes provoqués chez la volaille par des virus aviaires et par le virus de la maladie de Newcastle, caractérisé en ce qu'un liquide contenant du virus de la maladie de Newcastle ayant un titre de $10^{9,8}$ à $10^{10,5}$ $DI_{50}$ d'embryon par ml, est éventuellement homogénéisé et conjointement avec un liquide contenant du reo virus inactivé ayant un titre de $10^{5,5}$ à $10^{7,5}$ $DI_{50}$ d'embryon par ml, le rapport en volume entre le liquide contenant du virus de la maladie de Newcastle et le liquide contenant du reo virus pouvant varier de 3:4 à 5:1, est ajouté sous vive agitation et/ou homogénéisation à une phase huileuse contenant comme constituants essentiels au moins une huile choisie parmi les huiles minérales paraffiniques légères (qualité FDA), les huiles végétales et les huiles minérales naphténiques, conjointement avec un ou plusieurs émulsionnants appropriés sous la forme de composés surfactifs non ioniques, qui sont des esters ou ester-éthers issus d'oxydes d'alcoylène et/ou d'alcools hexahydroxylés et/ou d'acides gras naturels supérieurs (10 à 20 atomes de carbone), étant entendu que le rapport en volume entre la phase aqueuse formée par les deux liquides viraux et la phase huileuse peut varier de 7:13 à 3:7, un agent de conservation étant éventuellement ajouté à la phase aqueuse.

13. Procédé suivant la revendication 12, caractérisé en ce que le reo virus est celui de la souche LZ 671.

14. Procédé suivant la revendication 13, caractérisé en ce que le rapport en volume du liquid contenant le virus de la maladie de Newcastle au liquide contenant le reo virus est d'environ 3:2.

15. Souche de reo virus LZ 671 déposée le 6 avril 1979 à la Collection de l'Institut Pasteur à Paris sous le n° I.092.

**Revendications pour l'etat Contractant: AT**

1. Procédé de préparation d'un vaccin de reo virus inactivé, caractérisé en ce qu'on prépare un liquide contenant du reo virus en cultivant une souche de reo virus aviaire LZ 671 déposée le 6 avril 1979 à la Collection de l'Institut Pasteur à Paris sous le n° I.092 dans un oeuf de poule embryonné ou dans une culture de fibroblastes d'embryon de poussin exempts d'agents pathogènes spéfiés sous la forme d'une culture en couche monocellulaire en flacon roulant ou d'une culture de cellules fixées sur des supports solides inertes, en collectant le virus cultivé, en l'inactivant, en le mettant en suspension dans une solution taponnée, éventuellement en l'homogénéisant et en l'ajoutant sous vive agitation et/ou homogénéisation à une phase huileuse.

2. Procédé suivant la revendication 1, caractérisé en ce qu'on cultive la souche de reo virus LZ 671 dans un culture des cellules de départ sous la forme d'une culture en couche monocellulaire en flacon roulant ou de cellules fixées sur des supports inertes solides dans un milieu de culture formé de milieu de base d'Eagle, éventuellement additionné de 5 à 15 parties en volume de sérum de veau, d'environ 0,22% en poids de bicarbonate de sodium et de 1 à 5 parties en volume d'une solution ou suspension d'un ou plusieurs antibiotiques, de préférence la pénicilline G, la streptomycine et la natamycine, on sépare le liquide de culture lorsque la culture de cellules est pratiquement confluante, on inocule la culture d'une suspension de virus, on la met à incuber à 37°C pendant 0,5 à 3 heures pour fixer le virus, on ajoute un milieu d'entretien consistant en au moins du milieu de base d'Eagle avec 4 à 6 parties en volume de sérum de veau, 3 à 6 parties en volume de bouillon au Tryptose phosphate (100 g par litre), 10 à 20 parties en volume de liquide amniotique de bovin et les additifs précités et on récolte le milieu et les cellules contenant du virus 0,5 à 3 jours après l'apparition de l'effet cytopathogène (C.P.E.).

3. Procédé suivant la revendication 1, caractérisé en ce qu'on ajoute comme inactivateur de la bêta-propiolactone en une concentration de 0,05 à 0,25% en poids (à calculer sur le poids de la phase aqueuse) au liquide contenant du virus.

4. Procédé suivant la revendication 1, caractérisé en ce qu'on mélange le liquide contenant du virus avec une phase huileuse comprenant comme constituants essentiels au moins une huile choisie parmi les huiles minérales paraffiniques légères (qualité FDA), les huiles végétales et les huiles minérales naphténiques, conjointement avec 2 à 20% en poids d'une ou plusieurs émulsionnants appropriés sous la forme de composés surfactifs non ioniques, qui sont des esters ou ester-éthers issus d'oxydes d'alcoylène et/ou d'alcools hexahydroxylés et/ou d'acides gras naturels supérieurs ($C_{10}$ à $C_{20}$).

5. Procédé suivant la revendication 1, caractérisé en ce que le rapport en volume de la phase aqueuse formée par le liquid viral à la phase huileuse peut varier de 7:13 à 3:7.

6. Procédé suivant la revendication 1, caractérisé en ce que le rapport en volume de la phase aqueuse formée par le liquide viral à la phase huileuse est d'environ 7:13.

7. Procédé suivant la revendication 1, caractérisé en ce qu'on part d'un liquide contenant du reo virus ayant un titre d'au moins $10^{5,5}$ à $10^{7,5}$ $DI_{50}$ d'embryon par ml.

8. Procédé suivant la revendication 1, caractérisé en ce qu'on part d'un liquide contentan du reo virus ayant un titre de $10^{6,0}$ $DI_{50}$ d'embryon par ml.

9. Procédé de préparation d'un vaccin combiné inactivé prêt à l'administration contre les symptômes provoqués chez la volaille par des virus aviaires et par le virus de la maladie de Newcastle, caractérisé en ce qu'un liquide contenant du virus de la maladie de Newcastle ayant un titre de $10^{9,8}$ à $10^{10,5}$ $DI_{50}$ d'embryon par ml, est éventuellement homogénéisé et conjointement avec un liquide contenant du reo virus inactivé ayant un titre de $10^{5,5}$ à $10^{7,5}$ $DI_{50}$ d'embryon par ml, le rapport en volume entre le liquide contenant du virus de la maladie de Newcastle et le liquide contenant du reo virus pouvant varier de 3:4 à 5:1, est ajouté sous vive agitation et/ou homogénéisation à une phase huileuse contenant comme constituants essentiels au moins une huile choisie parmi les huiles minérales paraffiniques légères (qualité FDA), les huiles végétales et les huiles minérales naphténiques, conjointement avec un ou plusieurs émulsionnants appropriés sous la forme de composés surfactifs non ioniques, qui sont des esters ou ester-éthers issus d'oxydes d'alcoylène et/ou d'alcools hexahydroxylés et/ou d'acides gras naturels supérieurs (10 à 20 atomes de carbone), étant entendu que le rapport en volume entre la phase aqueuse formée par les deux liquides viraux et la phase huileuse peut varier de 7:13 à 3:7, un agent de conservation étant éventuellement ajouté à la phase aqueuse.

10. Procédé suivant la revendication 9, caractérisé en ce que le reo virus est de la souche LZ 671.

11. Procédé suivant les revendications 9 et 10, caractérisé en ce qu'on ajoute comme inactivateur de la bêta-propiolactone en une concentration de 0,05 à 0,25% en poids (à calculer sur le poids de la phase aqueuse) au liquide contenant le virus.

12. Procédé suivant les revendications 9 et 10, caractérisé en ce qu'on part d'un liquide de NDV ayant un titre de $10^{10}$ $DI_{50}$ d'embryon par ml.

13. Procédé suivant les revendications 9 et 10, caractérisé en ce qu'on part d'un liquide de reo virus LZ 671 ayant un titre $\geqslant 10^{6,0}$ $DI_{50}$ d'embryon par ml.

14. Procédé suivant les revendications 9 et 10, caractérisé en ce que le rapport en volume du liquide contenant du virus de la maladie de Newcastle au liquide contenant du reo virus LZ 671 est d'environ 3:2.

**Patentansprüche für die Vertragsstaaten: CH(LI), DE, GB, IT, NL, SE**

1. Inaktivierte Vakzine für eine Verwendung gegen Abweichungen im Skelett, wie rachitisähnliche Symptome, Oesteoporose c.q. Oberschenkeldegeneration verursacht durch Vogel-Reo-Virus allein oder in Kombination mit anderen, bei Geflügel, dadurch gekennzeichnet, daß sie von einer wässerigen Reo-Virus-Flüssigkeit abgeleitet ist, die durch Züchtung des LZ 671-Stammes, wie er am 6. April 1979 in der Collection d'Institut Pasteur, Paris, unter der Nummer I.092 hinterlegt worden ist, erhalten wurde.

2. Inaktivierte Vakzine für eine Verwendung gegen durch Vogel-Reo-Viren bei Geflügel hervorgerufene Diarrhöe und Waschstumshemmung, dadurch gekennzeichnet, daß sie von einer wässerigen Reo-Virus-Flüssigkeit abgeleitet ist, die durch Züchtung des LZ 671-Stammes, wie er am 6. April 1979 in der Collection d'Institut Pasteur, Paris, unter der Nummer I.092 hinterlegt worden ist, erhalten wurde.

3. Kombinierte, inaktivierte Vakzine, gebrauchsfertig gegen durch Vogel-Reo-Viren und durch das Newcastle Disease-Virus (NDV) bei Geflügel verursachte Symptome, dadurch gekennzeichnet, daß die Vakzine eine inaktiviertes Newcastle Disease-Virus enthaltende Flüssigkeit, die einen Titer von $10^{9,8}$ bis $10^{10,5}$ $EID_{50}$/ml aufweist, und eine inaktiviertes Reo-Virus enthaltende Flüssigkeit mit einem Titer von $10^{5,5}$ bis $10^{7,5}$ $EID_{50}$/ml, wobei das Volumsverhältnis zwischen der das Newcastle Disease-Virus enthaltenden Flüssigkeit und der das Reo-Virus enthaltenden Flüssigkeit von 3:4 bis 5:1 schwanken kann, und eine Ölphase enthält, die als wesentliche Komponenten mindestens ein Öl, ausgewählt aus Leichtparaffinmineralölen (FDA-Qualitat), Pflanzenölen und naphthenischen Mineralölen, zusammen mit zwischen 2 und 20 Gew.-% eines oder mehrerer geeigneter Emulgatoren in Form von nicht-ionischen, oberflächenaktiven Verbindungen, die Ester oder Ester-Äther abgeleitet von Alkylenoxiden und/oder 6-wertigen Alkoholen und/oder höheren natürlichen Fettsäuren (10 bis 20 Kohlenstoffatome) sind, aufweist, wobei das Volumsverhältnis zwischen der durch beide Virusflüssigkeiten gebildeten wässerigen Phase und der Ölphase von 7:13 bis 3:7 betragen kann.

4. Kombinierte, inaktivierte Vakzine nach Anspruch 3, dadurch gekennzeichnet, daß die wässerige Reo-Virus-Flüssigkeit durch Züchtung des LZ 671-Stammes, wie er am 6. April 1979 in der Collection d'Institut Pasteur unter der Nummer I.092 hinterlegt worden ist, erhalten wird.

5. Inaktivierte Vakzine nach Anspruch 1, dadurch gekennzeichnet, daß sie eine ölige Phase enthält, die als wesentliche Komponente mindestens eine Öl, ausgewählt aus Leichtaraffinmineralölen (FDA-Qualität), Pflanzenölen und naphthenischen Mineralölen, zusammen mit 2 bis 20 Gew.-% eines oder mehrerer geeigneter Emulgatoren in Form von nicht-ionischen, oberflächenaktiven Verbindungen, die Ester oder Ester-Äther abgeleitet von Alkylenoxiden und/oder 6-wertigen Alkoholen und/oder höheren natürliche Fettsäuren (10 bis 20 Kohlenstoffatome) sind, enthält.

6. Inaktivierte Vakzine nach Anspruch 5, dadurch gekennzeichnet, daß das Volumsverhältnis der von der Virusflüssigkeit gebildeten

11

wässerigen Phase und der öligen Phase von 7:13 bis 3:7 variieren kann.

7. Inaktivierte Vakzine nach Anspruch 6, dadurch gekennzeichnet, daß das Volumsverhältnis der durch die Virusflüssigkeit gebildeten wässerigen Phase und der öligen Phase etwa 7:13 beträgt.

8. Kombinierte, inaktivierte Vakzine nach Anspruch 3, dadurch gekennzeichnet, daß das Volumsverhältnis der Flüssigkeit, die das Newcastle Disease-Virus enthält, und der das Reo-Virus enthaltenden Flüssigkeit etwa 3:2 beträgt.

9. Verfahren zur Herstellung einer inaktivierten Reo-Virus-Vakzine, gekennzeichnet durch die Herstellung einer Reo-Virus enthaltenden Flüssigkeit durch Züchten eines Vogel-Reo-Virus-Stammes LZ 671, hinterlegt am 6. April 1979 in der Collection d'Institut Pasteur, Paris, unter der Nummer I.092, in einem bebrüteten Hühnerei oder in einer SPF-Hühnerembryo-Fibroblasten-Zellkultur in Form einer Monolayer-Rollkultur oder von Zellen, die an feste, inerte Träger gebunden sind, Sammeln des gezüchteten Virus, Inaktivieren und Suspendieren desselben in einer gepufferten Lösung, gewünschtenfalls Homogenisieren desselben, und Zusatz desselben unter kräftigem Rühren und/oder Homogenisieren zu einer öligen Phase.

10. Verfahren nach Anspruch 9, dadurch gekennzeichnet, daß als Inaktivator $\beta$-Propiolacton, gewünschtenfalls mit einem Stabilisator, zugesetzt wird.

11. Verfahren nach den Ansprüchen 9 und 10, dadurch gekennzeichnet, daß als Inaktivator $\beta$-Propiolacton in einer Konzentration von 0,05 bis 0,25 Gew.-% (bezogen auf das Gewicht der wässerigen Phase) zu der das Virus enthaltenden Flüssigkeit zugesetzt wird.

12. Verfahren zur Herstellung einer kombinierten, inaktivierten, gebrauchsfertigen Vakzine gegen durch Vogel-Reo-Virus und durch Newcastle Disease-Virus bei Geflügel verursachte Symptome, dadurch gekennzeichnet, daß eine Newcastle Disease-Virus enthaltende Flüssigkeit, die einen Titer von $10^{9,8}$ bis $10^{10,5}$ $EID_{50}$/ml hat, gewünschtenfalls homogenisiert wird und zusammen mit einer inaktivierten Reo-Virus enthaltenden Flüssigkeit, die einen Titer von $10^{5,5}$ bis $10^{7,5}$ $EID_{50}$/ml hat, wobei das Volumsverhältnis zwischen der Newcastle Disease-Virus enthaltenden Flüssigkeit und der Reo-Virus enthaltenden Flüssigkeit von 3:4 bis 5:1 betragen kann, unter kräftigem Rühreun-und/oder Homogenisieren einer öligen Phase zugesetzt wird, die als wesentliche Komponenten mindestens ein Öl, ausgewählt aus Leichtparaffinmineralölen (FDA-Qualität), Pflanzenölen und naphthenischen Mineralölen, zusammen mit einem oder mehreren geeigneten Emulgatoren in Form von nicht-ionischen, oberflächenaktiven Verbindungen, die Ester oder Ester-Äther abgeleitet von Alkylenoxiden und/oder 6-wertigen Alkoholen und/oder höheren natürlichen Fettsäuren (10 bis 20 Kohlenstoffatome) sind, enthält, wobei das Volumsverhältnis zwischen der von beiden Virusflüssigkeiten gebildeten wässerigen Phase und der Ölphase von 7:13 bis 3:7 schwanken kann, und gewünschtenfalls ein Konservierungsmittel zu der wässerigen Phase zugesetzt wird.

13. Verfahren nach Anspruch 12, dadurch gekennzeichnet, daß das Reo-Virus von Stamm LZ 671 ist.

14. Verfahren nach Anspruch 13, dadurch gekennzeichnet, daß das Volumsverhältnis der NDV enthaltenden Flüssigkeit und der Reo-Virus enthaltenden Flüssigkeit etwa 3:2 beträgt.

15. Reo-Virus-Stamm LZ 671, hinterlegt am 6. April 1979 in der Collection d'Institut Pasteur, Paris, unter der Nummer I.092.

**Patentansprüche für olen Vertragsstaat: Österreich**

1. Verfahren zur Herstellung einer inaktivierten Reo-Virus-Vakzine, gekennzeichnet durch die Herstellung einer Reo-Virus enthaltenden Flüssigkeit durch Züchten eines Vogel-Reo-Virus-Stammes LZ 671, hinterlegt am 6. April 1979 in der Collection d'Institut Pasteur, Paris, unter der Nummer I.092, in einem bebrüteten Hühnerei oder in einer SPF-Hühnerembryo-Fibroblasten-Zellkultur in Form einer Monolayer-Rollkultur oder von Zellen, die an feste, inerte Träger gebunden sind, Sammeln des gezüchteten Virus, Inaktivieren und Suspendieren desselben in einer gepufferten Lösung, gewüschtenfalls Homogenisieren desselben, und Zusatz desselben unter Kräftigem Rühren und/oder Homogenisieren zu einer öligen Phase.

2. Verfahren nach Anspruch 1, dadurch gekennzeichnet, daß der Reo-Virus-Stamm LZ 671 in einer Zellkultur in Form einer Monolayer-Rollkultur oder von Zellen, die an feste, inerte Träger gebunden sind, in einem Kulturmedium aus Eagle's Basalmedium gezüchtet wird, dem gewünschtenfalls 5 bis 15 Vol.-Teile Kalbsserum, etwa 0,22 Gew.-% Natriumbicarbonat und 1 bis 5 Vol.-Teil einer Lösung oder Suspension von einem oder mehreren Antibiotika, vorzugsweise Penicillin G, Streptomycin und Natamycin, zugesetzt sind, die Kulturflüssigkeit abgetrennt wird, wenn die Zellkultur fast geschlossen ist, mit Virussuspension angeimpft wird, bei 37°C 0,5 bis 5 h lang bebrütet wird, um das Virus anzulagern, und ein Aufrechterhaltungsmedium, bestehend aus zumindest Eagle's Basalmedium mit 4 bis 6 Vol.-Teilen Kalbsserum, 3 bis 6 Vol.-Teilen Tryptosephosphatbrühe (100 g/l), 10 bis 20 Vol.-Teilen Rinderamnionflüssigkeit und den oben erwähnten Zusatzstoffen, zugefügt wird, und das Virus enthaltende Medium und Zellen nach 0,5 bis 3 Tagen nach dem Auftreten des cytophatogenen Effekts (CPE) geerntet werden.

3. Verfahren nach Anspruch 1, dadurch gekennzeichnet, daß als Inaktivator $\beta$-Propiolacton in einer Konzentration von 0,05 bis 0,25 Gew-% (bezogen auf das Gewicht der wässerigen Phase) zu der das Virus enthaltenden Flüssigkeit zugesetzt wird.

4. Verfahren nach Anspruch 1, dadurch gekennzeichnet, daß die Virus enthaltende Flüssigkeit mit einer öligen Phase vermischt wird, die als wesentliche Komponenten mindestens ein Öl, ausgewählt aus Leichtparaffinmineralölen (FDA-Qualität), Pflanzenölen und naphthenischen Mineralölen, zusammen mit zwischen 2 und 20 Gew.-% eines oder mehrerer Emulgatoren in Form von nicht-ionische, oberflächenaktiven Verbindungen, die Ester oder Ester-Äther abgeleitet von Alkylenoxiden und/oder 6-wertigen Alkoholen und/oder höheren natürlichen Fettsäuren ($C_{10}$ bis $C_{20}$) sind, enthält.

5. Verfahren nach Anspruch 1, dadurch gekennzeichnet, daß das Volumsverhältnis der durch die Virusflüssigkeit gebildeten wässerigen Phase und der öligen Phase von 7:13 bis 3:7 variieren kann.

6. Verfahren nach Anspruch 1, dadurch gekennzeichnet, daß das Volumsverhältnis der durch die Virusflüssigkeit gebildeten wässerigen Phase und der öligen Phase etwa 7:13 beträgt.

7. Verfahren nach Anspruch 1, dadurch gekennzeichnet, daß von einer Reo-Virus enthaltenden Flüssigkeit mit einem Titer von mindestens $10^{5,5}$ bis $10^{7,5}$ $EID_{50}$/ml ausgegangen wird.

8. Verfahren nach Anspruch 1, dadurch gekennzeichnet, daß von einer Reo-Virus enthaltenden Flüssigkeit mit einem Titer von $10^{6,0}$ $EID_{50}$/ml ausgegangen wird.

9. Verfahren zur Herstellung einer kombinierten, inaktivierten, gebrauchsfertigen Vakzine gegen durch Vogel-Reo-Virus und durch Newcastle Disease-Virus bei Geflügel verursachte Symptome, dadurch gekennzeichnet, daß eine Newcastle Disease-Virus enthaltende Flüssigkeit, die einen Titer von $10^{9,8}$ bis $10^{10,5}$ $EID_{50}$/ml hat, gewünschtenfalls homogenisiert wird und zusammen mit einer inaktivierten Reo-Virus enthaltenden Flüssigkeit, die einen Titer von $10^{5,5}$ bis $10^{7,5}$ $EID_{50}$/ml hat, wobei das Volumsverhältnis zwischen der Newcastle Disease-Virus enthaltenden Flüssigkeit und der Reo-Virus enthaltenden Flüssigkeit von 3:4 bis 5:1 betragen kann, unter kräftigem Rühren und/oder Homogenisieren einer öligen Phase zugesetzt wird, die als wesentliche Komponenten mindestens ein Öl, ausgewählt aus Leichtparaffinmineralölen (FDA-Qualität), Pflanzenölen und naphthenischen Mineralölen, zusammen mit einem oder mehreren geeigneten Emulgatoren in Form von nicht-ionischen, oberflächenaktiven Verbindungen, die Ester oder Ester-Ather abgeleitet von Alkylenoxiden und/oder 6-wertigen Alkoholen und/oder höheren natürlichen Fettsäuren (10 bis 20 Kohlenstoffatome) sind, enthält, wobei das Volumsverhältnis zwischen der von beiden Virusflüssigkeiten gebildeten wässerigen Phase und der Ölphase von 7:13 bis 3:7 schwanken kann, und gewünschtenfalls ein Konservierungsmittel zu der wässerigen Phase zugesetzt wird.

10. Verfahren nach Anspruch 9, dadurch gekennzeichnet, daß das Reo-Virus vom Stamm LZ 671 ist.

11. Verfahren nach Anspruch 9 und 10, dadurch gekennzeichnet, daß als Inaktivator $\beta$-Propiolacton in einer Konzentration von 0,05 bis 0,25 Gew.-% (bezogen auf das Gewicht der wässerigen Phase) zu der Virus enthaltenden Flüssigkeit zugesetzt wird.

12. Verfahren nach Anspruch 9 und 10, dadurch gekennzeichnet, daß von einer NDV-Flüssigkeit mit einem Titer von $10^{10}$ $EID_{50}$/ml ausgegangen wird.

13. Verfahren nach Anspruch 9 und 10, dadurch gekennzeichnet, daß von einer LZ 671 Reo-Virus-Flüssigkeit mit einem Titer von $\geqslant 10^{6,0}$ $EID_{50}$/ml ausgegangen wird.

14. Verfahren nach Anspruch 9 und 10, dadurch gekennzeichnet, daß das Volumsverhältnis der das Newcastle-Virus enthaltenden Flüssigkeit und der das LZ 671 Reo-Virus enthaltenden Flüssigkeit etwa 3:2 beträgt.